# EUROPEAN PATENT APPLICATION

(11) **EP 4 321 204 A1**
(43) Date of publication of application: **14.02.2024**
(21) Application number: 22382775.9
(22) Date of filing: 10.08.2022
(51) Int. Cl.: A61M 39/02

(54) **SUBCUTANEOUS VASCULAR ACCESS DEVICES AND METHODS**

(71) Applicant: Krauel Giménez-Salinas, Lucas, 08017 Barcelona (ES)
(72) Inventor: Krauel Giménez-Salinas, Lucas, 08017 Barcelona (ES)
(74) Representative: de Rooij, Mathieu Julien

(57) **Abstract**

The present disclosure relates to implantable vascular access devices. The present disclosure further relates to methods and kits including such vascular access devices and delivery systems for delivering such devices to a suitable implantation site. The present disclosure also relates to methods for implanting such devices and methods for locating such devices once implanted.

## Description

### TECHNICAL FIELD

The present disclosure relates to implantable medical devices, and more particularly relates to implantable vascular access devices. The present disclosure further relates to methods for locating and accessing implanted vascular access devices, and to methods for implanting and positioning such devices.

### BACKGROUND

Vascular access systems are known to be used to provide recurring access to the body of a patient during treatment or for diagnostic purposes. For example, the use of such implants is known in oncology treatments. The implant typically contains a port which is closed off by a septum and a catheter that is connected to the port. The port may be implanted in the chest area of a patient in a "pocket" created underneath the skin of the patient.

The catheter may be connected to the patient's jugular vein, subclavian vein or other major vein draining into the vena cava (usually superior vena cava). A needle may be used to inject a therapeutic substance e.g. chemotherapeutic agents into the port to deliver it to the body. Also, a needle may be used to extract blood from the patient for diagnostic or analytical purposes from shallow tissue areas in the body of the patient, such as subcutaneously under the skin.

Even though the port may be rather small, depending on a patient's age and body type, a port may be seen as an area protruding from a patient's chest.

A significant incision e.g., about 2 cm or 2,5 cm, has to be made to create the subcutaneous pocket to insert the implant.

In order to perform an injection, a medical professional will rely on palpation techniques to locate the port. Even if the port has been located, still the septum which is to be punctured for access cannot be seen by the professional. A commonly used technique is to press down on the port to make sure the port does not move during or prior to injection.

In order to enhance procedures for locating and accessing ports, it is known to provide ports with multiple small protrusions. Recognition of these protrusions can reduce errors when inserting the needles. WO 2007/041471 provides a plurality of LEDs and light guides to provide a plurality of visual indicators around the septum of the port to aid in accessing the port. The LEDs may be activated by pushing on the port. Such devices have been commercialized but have been withdrawn from the market as they create significant side effects related to extrusion and infection of the device.

DE 10 2015 122 061 discloses a port with a plurality of light modules.

Complications with the use of different ports include large scars, surgical wound infections and extrusions of the device. Other common complications include wrong injections (i.e. not in the port but next to the port). This can lead to pain, and anxiety for the patient, increased risk of catheter infection, and bruising. This can be particularly traumatic for pediatric patients.

An undetected access error results in medication being infused outside the reservoir (subcutaneous cellular tissue) with serious consequences in the case of very irritating drugs that can end in skin necrosis and the need for removing the device and substituting it for another. Access errors exponentially increase the risk of infection since more frequent handling leads to a greater risk of infection. The infection of a port is a serious complication (catheter sepsis) that often does not respond to antibiotic treatments. When this occurs, the device may need to be removed following a surgical procedure in an operating room generally accompanied by local or general anesthesia. The surgical procedure in pediatric patients requires general anesthesia, which may also increase patient anxiety. Moreover, repeated general anesthesia procedures increases the risk of neurological disorders.

Moreover, the use of ports can be uncomfortable and even traumatic for patients because of aesthetic aspects, discomfort during sports, and even normal activities (e.g. putting on a seatbelt of a car).

The present disclosure aims to provide methods and devices that avoid or at least reduce one or more of the aforementioned disadvantages.

### SUMMARY

In an aspect of the disclosure, an implantable vascular access device is provided. The device comprises a housing, a reservoir with an exit lumen, a septum for closing the reservoir and defining an injection area, a visualization system for visualizing the injection area and a controller configured to activate the visualization system. The implantable vascular access device is self-dissecting.

In accordance with this aspect, an implantable vascular access device is provided which is self-dissecting. Self-dissecting as used throughout the present disclosure may be understood to refer to a device that is shaped and sized such that it does not require specific dissecting to create space for implanting or positioning. Whereas prior art ports require the creation of a pocket underneath the skin and generally underneath the subcutaneous adipose tissue, the vascular access device according to this aspect does not require such a pocket. The ability to be introduced underneath the subcutaneous tissue without the need for dissecting is generally a combination of size and shape. In particular, the dimensions of the implantable vascular device may be generally smaller than prior art devices and in examples, the outer shape of the vascular access device may have a tapered portion. Along the tapered portion, width and/or height may gradually increase to facilitate gradual introduction. Bumps, steps, protrusions may generally be avoided along the outer surface, and specifically along the tapered portion.

In examples, the vascular access device may be significantly smaller than hitherto known ports. Since the vascular access device according to this aspect is significantly smaller, it is generally not visible from an outside and is more comfortable to patients. When the device is to be located, e.g. when an injection is to be made, or a blood sample is to be withdrawn, the device can however not be easily palpated or seen from the outside. In this respect, the vascular access device according to this aspect includes a visualization system, and a controller to activate the visualization system to aid a professional (doctor, surgeon, nurse) to locate the device, such that palpation is not necessary.

According to a second aspect, an implantable vascular access device is provided, which comprises a housing, a reservoir with an exit lumen, a septum for closing the reservoir and defining an injection area and one or more sensors arranged to measure one or more parameters of blood in the reservoir. The implantable vascular access device further includes a transmitter for sending the measured parameters. In accordance with this aspect, an implantable vascular access device is provided which can be used to obtain measurements remotely. Since the implantable vascular access device is in direct communication with a patient's vessels, measurements can be made by the sensors and then the data obtained may be transmitted. There will thus be no need or less of a need for a professional to puncture the patient to obtain a sample for analysis. The patient experience may thus be less traumatic, specifically for children.

In examples, the sensors may be configured to measure one or more of: a temperature, cardiac rhythm, oxygen saturation, glycaemia, pH, lactate and others.

The first and second aspects may also be combined.

In some examples, a height of the device may be less than 10 mm, specifically less than 8 mm, and more specifically 6 mm or less. Since the device does not need to be palpated for location, the device can be made as small as possible, and specifically as "flat" as possible. In examples, the height of the device may depend on the size of the catheter attached to the port, and the height of the device may just be the height required in order to couple the catheter.

In some examples, the injection area may have a size of 75 mm2 or more, specifically 85 mm2 or more, more specifically 95 mm2 or more. Even though it is generally desirable to reduce the dimensions of the vascular access device as much as possible, the most important dimension in terms of patient comfort is likely the height of the vascular access device. In order to make injections safer, and/or in order to make the device more durable, the injection area may actually be larger than in the prior art. Increasing the injection area also reduces the repetition of needle puncturing in the same location and thus can reduce scarring.

In some examples, a width of the vascular device may be 12 mm or less, specifically 10 mm or less, and more specifically 8 mm or less or even 5mm or less. A reduction of the width may reduce the size of the injection area. The smallest width for a commercially available pediatric vascular access device nowadays is 19mm. A width of 12 mm allows for a skin incision of 10mm or even smaller. This dimension allows as well for tunneling and self-dissection with specific tunneler as will be disclosed hereinafter.

A width of 8 mm or smaller is desired for a smaller skin incision and even easier tunneling. A width of 5 mm or smaller even further improves this and can be achieved e.g. by increasing the length of the device as this will maintain or can even augment the injection area.

As the device according to examples of the present disclosure is generally not noticeable under the skin thanks to its limited height and visualization system, the dimensions can vary. Specifically a length of the vascular access device may be increase to increase the size of the injection area without negatively affecting performance otherwise.

In examples, a length of the vascular access device may be 30 mm or more, specifically 35 mm or more, more specifically 40 mm or more, or even 50 mm or more. In examples, the vascular access device according to the present disclosure may be significantly longer than in the prior art. An increase in length of the vascular access device may particularly be provided by a tapered portion of significant length. The tapered portion may have a tapering height and/or a tapering width.

In examples, the visualization system may be configured to indicate a perimeter of the injection area. The visualization system may be arranged e.g. along a perimeter of the injection area. A nurse or other professional then simply needs to make sure to puncture within the perimeter. Errors during injection and corresponding complications for the patient may be avoided or reduced.

In examples, the visualization system may be configured to indicate a puncture site within the injection area i.e. some indication is provided to the medical professional where, within the injection area, a puncture should preferably be performed. Suitable puncture sites may be varied in order to avoid wear or degradation of a specific portion of the septum.

In some examples, the visualization system comprises one or more light emitting devices arranged along a perimeter of the injection area. Suitable light emitting diodes may be used to transmit light upon activation and the light transmitted may be visible through the skin of the patient. A plurality of LED's may be arranged along a perimeter of the injection area. In examples, the LED's used may be millimetric (width or diameter of the LEDs may be e.g. 1 - 3 mm). A plurality of LEDs can be used to mark a border of the injection area. Alternatively, a ring shaped LED may be used to indicate the perimeter of the injection area. The use of a plurality of light emitting devices such as LEDs can also be used to spread needle punctures over the injection area.

In further examples, the visualization system comprises an augmented reality device. A nurse (or other professional) may wear suitable glasses with an augmented reality system. When the visualization system is activated, the location of the vascular access device (or specifically the injection area) may be projected onto the patient's skin, such that puncturing the septum of the device is facilitated.

In examples, the device may further comprise an energy source, optionally a battery. In other examples, power/energy may be supplied remotely e.g. upon activation by another device. E.g. wireless power transmission may be used.

In examples, the controller may be configured to activate the visualization system after receiving an instruction from a remote control. Such a remote control may e.g. be a PDA, tablet, or a Smartphone. When the device needs to be accessed, a remote control may send instructions to the controller to activate the visualization system. The visualization may be programmed in a variety of ways, and may be programmed to illuminate the injection area (or a perimeter thereof) during a predefined period of time, which may be e.g. 1 minute or two minutes, or any other amount of time that is suitable for the procedure to be completed. After the predefined period of time, the visualization system may be turned off. Power consumption may thus be suitably reduced.

The same PDA, tablet or Smartphone may also be used to obtain measured variables from the sensors (if available).

In some examples, the exit lumen is provided at a first end of the device, and the device may further comprise a device side connector for releasably coupling to a connector of a delivery system. The vascular access device may be pulled or dragged towards a suitable site.

In some examples, the device side connector may be an eye or eyelet configured to receive a hook of the delivery system. In other examples, the device side connector is a connector for magnetically coupling to the connector of the delivery system.

In a further aspect, a system is provided, which comprises a vascular access device according to any of the examples, and further comprises a data processing system comprising the receiver for receiving the data sent by the transmitter of the device and a database for storing the received data.

In examples, such a system may further comprise a display for visualizing the received data. In examples, the data processing system may be a personal computer, a tablet, a mobile phone, a Smartphone, or a personal digital assistant. Any other suitable data processing system may be used.

In examples, the data processing system may not only receive the data from sensor and process them further, or transmit them for further processing, the data processing system may also be used for sending instructions to the vascular access device. E.g. instructions for measurement and/or instructions for activation of the visualization system may be sent from such a system.

In some examples, the data processing system may store the data for further processing and/or upload data to a portal of a hospital or another website. Patients and/or other healthcare professionals may consult the data when necessary. In further examples, data may be sent to a Smartphone of a patient or guardian or representative of the patient. Users may download a suitable application to consult, and visualize the data.

In yet a further aspect, a kit is provided, which comprises the implantable vascular access device according to any of the herein disclosed examples, and a remote control for sending one or more instructions to the controller of the implantable vascular access device.

The remote control may be a personal computer, a tablet, a mobile phone, a Smartphone, or a personal digital assistant. Such a remote control may be used to send instructions, request measurement data if sensors are provided, activate the visualization system or other. In some examples, the remote control may be integrated in a needle. A visualization system may be activated due to proximity of the needle.

In yet a further aspect, a method for locating a vascular access device implanted in a patient is provided. The method comprises remotely activating a visualization system of the vascular access device to visualize a perimeter of an injection area of the vascular access device, and locating the vascular access device by detecting the perimeter of the injection area while refraining from palpating the patient.

In accordance with this aspect, a vascular access device and the injection area of such a device may be identified to a professional without palpation. This allows the vascular access device to be generally smaller and, in particular, flatter than prior art devices.

In examples, the vascular access device may not be visible at all from an outside of the patient when the visualization system is not activated. In examples, the vascular access device is made to be practically impossible to locate through palpation.

In yet a further aspect, a delivery system for delivering a vascular access device is provided. The vascular access device has a first end and a second end, and an exit lumen with a device side connector for coupling to a catheter arranged at the first end. The delivery system comprises a tunneler configured to provide a tunnel between a first access site and a second access site, wherein the tunneler is further configured to be releasably coupled to the vascular access device at the second end of the vascular access device.

In accordance with this aspect, a delivery system is provided which can implant a vascular access device to a suitable implantation site without the need for a creation of a pocket. A delivery system is herewith provided that is specifically suitable for the implantation of a vascular access device of generally reduced dimensions. The tunnel created subcutaneously may be occupied by the catheter attached to the vascular access device. Tissue growth around the catheter can occur afterwards.

The first access site herein may be regarded as the venous access site. The second access site is comparable to the device access site in the prior art. However, in examples of the present disclosure no such classical device access site is necessary.

Throughout the present disclosure, tunneling may be understood as the creation of a subcutaneous passageway. A tunneler, also known as "vascular tunneler" or "tunneller" is a device that is configured for tunneling. A tunneler may generally comprise a tubular body and a "tunneling" tip, wherein the tip separates the tissue to create the tunnel.

In examples, the tunneler may be configured to release the vascular access device in a location between the first access site and the second access site. The tunneler may be used to pull the vascular access device from the first access site to the second access site and then release the vascular access device at the intended location.

The first access site may be a vascular access point, i.e. in or near a neck or infraclavicular region of the patient. The second access site may be in an armpit region of a patient. Since no port is created and only very small incisions are required, only a small scar will remain and thus suitable access sites may be chosen with less regard for aesthetic appearances.

In yet a further aspect, a method for delivering a vascular access device to an implantation site is provided. The method comprises providing a first access site through a skin of a patient, introducing a catheter in a vein through the first access site, providing a second access site through the skin of the patient and providing a tunnel between the first access site and the second access site using a tunneler. The method further comprises releasably coupling the tunneler to the vascular access device, and moving the tunneler from the first access site towards the second access site to pull the vascular access device towards the second access site and releasing the vascular access device in the implantation site.

In some examples, providing the tunnel between the first access site and the second access site and pulling the vascular access device towards the second access site may be performed simultaneously. I.e. a tunnel is created from the first access site to the second access site, and at the same time, the vascular access device (which has the catheter coupled to it) is dragged to the desired site.

In other examples, a tunnel may be provided from the second access site to the first access site using the tunneler. Subsequently the tunneler may be releasably coupled to the vascular access device (including catheter), and the tunneler may be moved from the first access site to the second access site.

In examples, providing the second access site comprises making an incision in the skin, wherein the incision may be smaller than 0,5 cm in length, specifically smaller than 3 mm in length, and more specifically 2 mm or smaller in length. In prior art systems and methods, a relatively large incision may be needed at the second access site (close to the implantation site of the port), because a sizable port is introduced there and a pocket is created. In examples of the present disclosure, the port may be significantly smaller, and only the tunneler may enter and exit the skin from the second access site. This can lead to less scarring.

In some examples, the tunneler may be releasably mechanically coupled to the vascular access device. In other examples, a magnetic coupling or further alternative may be used.

In some examples, the method may comprise visualization of the vascular access device and/or the catheter while the vascular access device is pulled from the first access site to the second access site. Optionally, visualization with X-rays of the catheter location in the vein may be used to ensure proper catheter placement.

### BRIEF DESCRIPTION OF THE DRAWINGS

Non-limiting examples of the present disclosure will be described in the following, with reference to the appended figures, in which:
Figures 1A - 1E schematically illustrate a first example of a vascular access device according to the present disclosure;
Figures 2A - 2C illustrate further examples of a vascular access device;
Figures 3A - 3C schematically illustrate an example of a delivery system of a vascular access device;
Figures 4A and 4B schematically illustrate an example of a method for implanting a vascular access device; and
Figure 4C illustrates an alternative example of a method for implanting a vascular device.

The figures refer to example implementations and are only be used as an aid for understanding the claimed subject matter, not for limiting it in any sense.

### DETAILED DESCRIPTION OF EXAMPLES

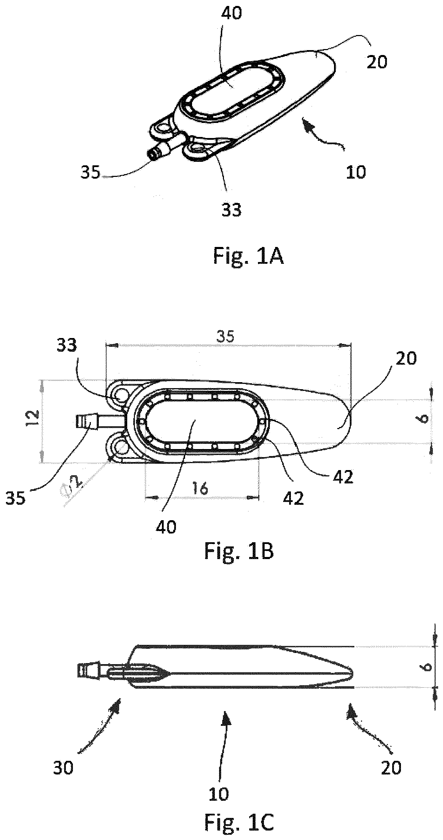

Figures 1A - 1E schematically illustrate an implantable vascular access device 10. The vascular access device 10 comprises a housing and a reservoir 69 with an exit lumen 37. The vascular access device further comprises a septum 67 for closing the reservoir 69 and defining an injection area 40.

The device 10 may further comprise a visualization system for visualizing the injection area 40 and a controller configured to activate the visualization system. The implantable vascular access device in this example is self-dissecting.

The injection area in this example is substantially obround. Other shapes and sizes are also envisaged for the injection area 40.

In this example, the visualization system comprises a plurality of light emitting devices arranged along a perimeter of the injection area 40. In this example, a plurality of LEDs 42 are arranged at a relatively short distance from each other such as to be able to illuminate and indicate the perimeter of the injection area. In some examples, the LEDs may have a width or diameter of e.g. 1 mm - 4 mm. A pitch of the LEDs may be e.g. 3 mm - 10 mm.

The device may further comprise an energy source, e.g. one or more batteries. The controller of the device 10 may be configured to activate the visualization system after receiving an instruction from a remote control. The controller may be configured to activate all LEDs 42 to indicate the injection area after implantation. The electromagnetic radiation emitted by the LEDs 42 may illuminate the skin of the patient, and thus the location of the device 10 becomes visible from the outside. In particular, it will be clear from the outside where an injection is to be made. An injection only has to be made within the illuminated injection area.

In examples, the LEDs 42 may be activated for a predetermined period of time, e.g. between 20 seconds and 2 minutes, or any time period that is determined to be sufficient for carrying out an injection, injecting a drug or extracting a blood sample. After the predetermined period of time, the LEDs 42 may be turned off.

In examples, the LEDs 42 may all be activated in the same way. In other examples, LEDs may illuminate light of different colors. For example, by using a different color for a selection of the LEDs, e.g. a preferred area for injection may be indicated within the injection area. One factor limiting the lifetime of an implanted vascular access device is the septum 67. The septum 67 may generally be made of a medical grade biocompatible silicon. Depending on the thickness and overall area of the injection area, a predetermined number of punctures may be made before the septum starts to degrade, and the vascular access device would have to be replaced. By spreading injections throughout the injections area (by subsequently indicating different zones within the injection area), the resulting scar from successive puncture may be less noticeable and the lifetime of the device may be extended. Or for a given required lifetime, the injection area may be smaller.

Although not shown in the illustrated example of figures 1A - 1E, other systems or methods for visualizing the location of the implanted vascular access device from the outside may be used. In alternative examples, the visualization system may comprise an augmented reality device. For example, a professional (e.g. doctor or nurse) may wear augmented reality glasses. The glasses may include a suitable camera system. The controller may activate markers (such as LEDs) arranged in the device 10 which may be registered by the suitable camera system such that a display in the glasses can help the professional towards the correct site for injection. In such a case, the markers do not need to be arranged at or along the perimeter of the injection area 40. As long as the augmented reality system can identify the markers, a visual representation of the vascular access device 10 or of the injection area 40 may be reproduced.

The vascular access device 10 may comprise a first end 30 and an opposite second end 20. The exit lumen 37 may be provided at the first end 30 of the device 10. At the first end 30, the device further comprises connector 35 for coupling to a catheter. The connector 35 may be made e.g. from titanium of plastic.

The catheter is not illustrated in figures 1A - 1E, but is schematically illustrated as catheter 80 in figures 1A - 2C and 3A -3B. Any catheter suitable for this purpose may be used. The vascular access device 10 may be used e.g. in combination with catheters in use nowadays for the same purpose. Diameters and lengths of the catheter may be the same as known in the art, and may depend specifically on the patient's anatomy. Suitable materials for catheters include e.g. silicone or polyurethane.

In examples, one or more eyelets 33 may be provided at the first end 30 of the device 10. The eyelets 33 may be used for suturing the device 10 to tissue at the implantation site. It is envisaged that the vascular access device 10 may be sized and shaped such that the risk of displacement or "flipping" of the vascular access device 10 is small, in particular in examples wherein no pocket is created for fitting the device. However, in some cases, as a precaution, extra sutures may be provided at the eyelets 33 to ensure the correct location and orientation of the device. In other examples, no such eyelets (or other features suitable for fixing or suturing the device in place) may be necessary.

The device may further comprise one or more sensors for measuring patient parameters. The sensors may be arranged near the second end 40 of the device 10. The sensors may be arranged on or in connection with an electronics carrier 66, herein depicted as a box for the purposes of simplification. The electronics carrier 60 may be arranged in a variety of positions. In the illustrated example, a cavity is formed in the housing at the second side 20 of the device 10. The housing may have a tapered portion which gradually decreases the height from the injection area to the end of the device 10. The cavity for fitting the electronics carrier 60 may be provided in this tapered portion.

The sensors may include e.g. sensors for measuring one or more of temperature, cardiac rhythm, oxygen saturation, and glycaemia. Depending on the type of sensor, the sensors may be arranged so as to be in contact with blood in the reservoir 69. Furthermore, a timer (e.g. for determining a timestamp of an injection, or a timestamp for activation of the visualization system), and communication systems may be included.

The electronics carrier 66 may be or comprise printed circuit board. The electronics carrier may include the logic herein described as the "controller" to active and control the visualization system. The electronics carrier 66 may include a receiver for receiving instructions, and may include an emitter to send data. In particular, sensor data may be transmitted to a receiver in a data processing system. The data processing system may be a tablet, a Smartphone, a general-purpose computer or otherwise. Communication with the vascular access device 10 may be based e.g. on NFC, Wi-Fi, Bluetooth or other. The electronics carrier 66 may also carry a power source like a battery.

The data processing system may process and/or analyze and/or store and/or visualize and/or forward or otherwise process the data. For example, the data may be stored in a hospital data storage, or may be sent onwards to a patient's device (e.g. Smartphone), and may be uploaded in a website portal for consultation by patients and/or medical professionals. Thus, medical records, health information, medication already given, or other patient relevant information may be stored and retrieved from the patient's device.

The data processing system may further be configured to send one or more instructions to the controller of the implantable vascular access device. Instructions may include a command to send data, a command for activating one or more LEDs, a command to communicate battery status and other. In a specific example, an operator may access a suitable application on a Smartphone and send instructions, e.g. illuminate for a fixed or manually changeable period of time.

In a further aspect, a kit is provided which comprises the implantable vascular access device according to any the examples disclosed herein, and a remote control for sending one or more instructions to the controller of the implantable vascular access device. The remote-control device may be the same data processing system that receives and processes sensor data. E.g. a Smartphone or tablet may be used both for sending instructions to the vascular access device to activate the visualization system, to send measurement data, to turn power on or otherwise. The same device may include a suitable application for receiving, storing and visualizing e.g. received sensor data. The same electronic device may even be programmed to generate a warning signal if the received sensor data indicates an anomaly. Such an anomaly may be a malfunctioning of the device or its sensors, or may be an anomaly in the data indicating a possible health issue of the patient.

The remote-control device may also be a different device. E.g. a remote control may be a dedicated device used for activation of the visualization system, whereas another electronic device is used for data collection.

The aforementioned warning signal may also be generated by the vascular access device when outside a hospital setting e.g., when the patient is at home. The vascular access device may be programmed to determine certain variables (oxygen saturation, hearth rhythm and others) at regular intervals. If an anomaly is detected, the warning signal may be sent to a medical professional and/or to a patient's electronic device.

In some examples, the electronics carrier may include a GPS or other localization system, such that the patient can be located in case a warning signal is sent.

In further specific examples, the remote control may be integrated in a needle. E.g. a needle device may first be brought into proximity of the patient and send a wakeup or activation command to the vascular access device 10 for illuminating the injection area 40.

Figures 1B an 1C illustrate possible dimensions of a vascular access device 10 according to one example. The vascular access device 10 may be smaller, and specifically flatter than prior art devices. The vascular access device 10 may also be narrower, i.e. have a smaller width than prior art devices.

A height of the device may be less than 10 mm, specifically less than 8 mm, and more specifically 6 mm or less. In some examples, the height of the device may be about the diameter of the catheter that is to be coupled to the device 10. In examples, the device 10 may be configured not to be palpable through a skin of the patient. The device 10 may be configured not to be visible from the outside.

In the example illustrated in figure 1B, a width of the device may be about 12 mm.

For comparison, one of the smallest available pediatric vascular access device available on the market has a height of 11 mm, and a width of 23 mm. This is so because even the smallest available pediatric device nowadays is designed to be palpable, whereas examples of the present disclosure do not rely on palpation for identifying the location of the device 10.

The device 10 may be shaped such as to have a generally smooth outer surface i.e. without sharp corners, or protrusions. Sharp corners, protrusions and the like may increase visibility from the outside and aid in locating the device using palpation, but at the same time can cause problems of irritating, infecting and protruding through the skin. In such a case, further inventions are necessary to substitute a vascular access device.

The injection area may also be made as small as possible. In other examples, the injection area may be larger than in the prior art, to compensate for any perceived risk by not relying on palpation for locating the device. The injection area may have an area of 75 mm² or more, specifically 85 mm² or more, more specifically 95 mm² or more. It should be noted that with the design illustrated in e.g. figures 1A - 1E, it is possible to reduce height and width of the vascular access device 10 and maintain substantially the same or even have an increased injection area as compared to prior art devices.

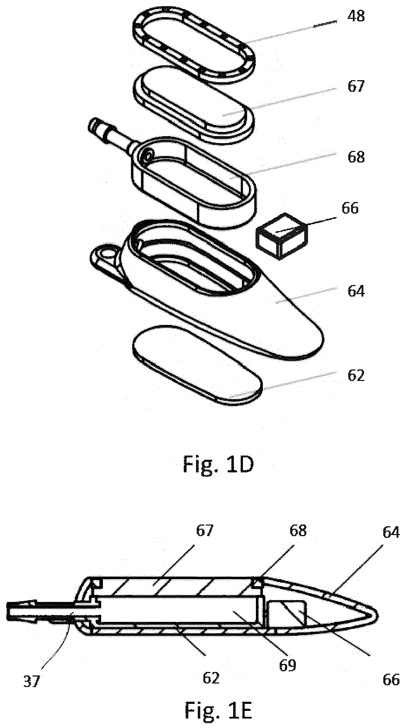

Figures 1D and 1E schematically illustrate a build-up of the components of the vascular access device according to the illustrated example. The septum 67 may have a recess along its perimeter. The septum 67 may have a smooth and substantially flat top surface, i.e. bumps or protrusions as used in some prior art devices may be avoided.

A ring-shaped electronic carrier strip 48 comprising a plurality of LEDs fits in the recess to be substantially flush with the top surface of the septum 67. The electronic carrier strip may be attached to the septum 67 and housing 64 using e.g. a suitable adhesive.

The vascular access device 10 may further comprise a puncture-proof bowl 68, which may be made of e.g. titanium or plastic. The bowl 68 may be fit in housing 64. The bowl 68 may have a through-hole for fitting the connector 35 for the catheter. The housing 64 may comprise a tapered portion at the second end. The vascular access device 10 may further comprise a bottom lid 62 to close off the device at a bottom side.

In other non-illustrated examples, the LEDs may be arranged in a different position, e.g. underneath bowl 68. The septum 67 and bowl may be transparent such that illumination by the LEDs may still be visible from the outside of the patient.

In further examples, the specific location of the LEDs with respect to the injection area and the number of LEDs may be varied if the injection area can clearly be identified from the outside of the patient's body. In examples, flashing of some of the LED's, varying colors or other signaling may be used to indicate a suitable puncture site to the medical professional, to that the needle punctures may be suitably (e.g. randomly) varied over the injection area.

A method for locating a vascular access device implanted in a patient may comprise remotely activating a visualization system of the vascular access device 10 to visualize a perimeter of an injection area 40 of the vascular access device 10. And the method comprises locating the vascular access device 10 by detecting the perimeter of the injection area 40 while refraining from palpating the patient.

In some examples, remotely activating the visualization system may comprise sending an activation signal from one or more of a personal computer, a tablet, a mobile phone, a Smartphone, or a personal digital assistant.

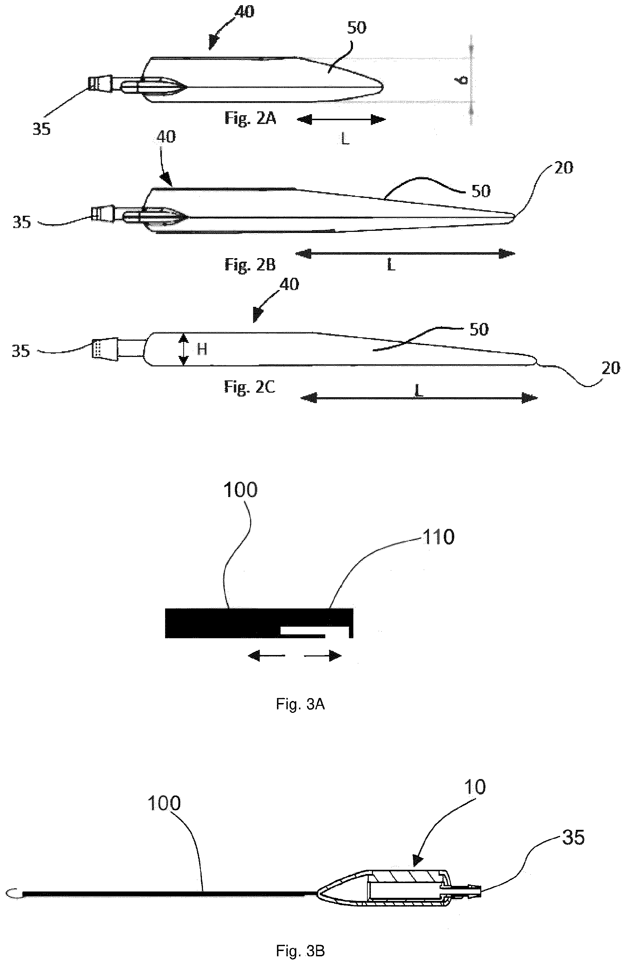

Figures 2A - 2C illustrate further examples of the vascular access device 10. The example of figure 2A corresponds substantially to the example of figure 1, and in particular the figure 2A corresponds to figure 1C.

Figures 2B and 2C illustrate two alternatives. A height of the two vascular access device is the same as in the example of figure 1, i.e. 6 mm. In figures 2B and 2C, a length of the tapered portion has been increased. A length L of the tapered portion in the example of figure 2A may be approximately 15 mm. A length of the tapered portion in the examples of figures 2B and 2C may be 30mm or more, specifically 40 mm or more. An increased length of the tapered portion makes the device more suitable for self-dissecting: the gradual increase of the height can facilitate the introduction of the device.

In the example of figure 2B, both the top surface and the bottom surface of the device taper towards the end 20. The height of the end 20 substantially corresponds to a middle of the device side connector 35 for coupling to a catheter.

In the example of figure 2C, the bottom surface is substantially flat. The top surface tapers towards the bottom.

In examples, the taper angle(s) of the tapered portion may be 35º or smaller, specifically 25º or smaller, and more specifically 15º or smaller.

Figures 3A - 3C schematically illustrate a delivery system for delivering a vascular access device such as the device 10 of figures 1A - 1E or any of the examples of figures 2A - 2C. The vascular access device may have a first end 30 and a second end 20, and an exit lumen 37 with a device side connector 35 for coupling to a catheter arranged at the first end 30. The delivery system comprises a tunneler 100 configured to provide a tunnel between a first access site and a second access site. The tunneler may further be configured to be releasably coupled to the vascular access device 10 at the second end 20 of the vascular access device, i.e. the opposite end of the end where the catheter 80 may be coupled.

The terminology of "first" and "second" access site do not imply any specific order of when the access is provided. That is, the first access site may be created before the second access site or *vice versa.*

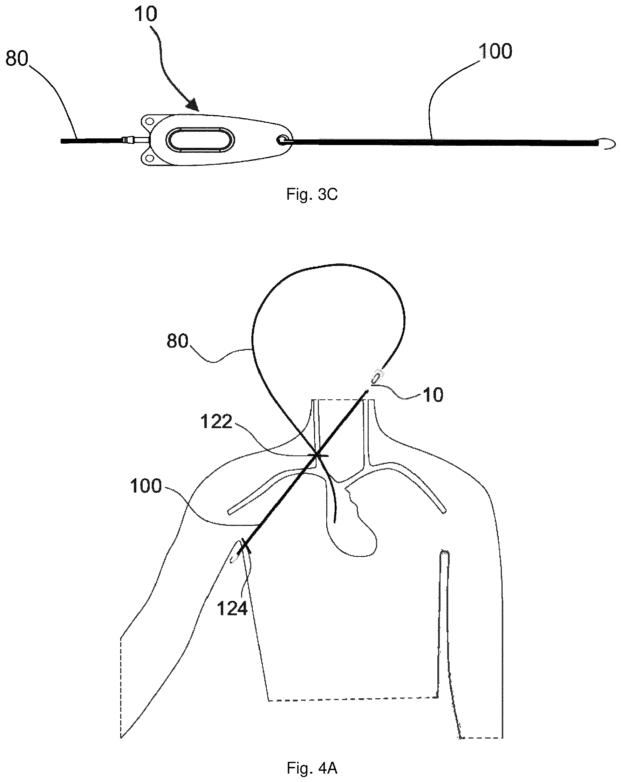

The first access site is the site where a catheter is originally inserted into a suitable vein, as will be explained in more detail with reference to figure 3. The first access site may be in or near a neck of patient and infraclavicular region of the patient. In order to position the catheter in the vein, the Seldinger technique may be used. Depending on the circumstances and on the systems used, the procedure may involve the puncturing of the skin with a hollow needle to position a catheter in the vein. Using one or more of sheaths, dilators and introductory catheters, the catheter that is to be attached to the vascular access device may be positioned in the vein. Other techniques to access the vein may also be used, such as a modified Seldinger technique, a cutdown procedure or an over-the-wire technique.

The second access site is the site for introduction or exit of the tunneler. The second access site may be closer to the implantation site of the vascular access device. In prior art methods and systems, the second access site usually requires an incision (and subsequent suturing) for dissection and creation of a pocket of sufficient size for the subsequent introduction of the vascular access device. Although examples of the vascular access device may be implanted in the same manner as in the prior art, in other examples, the vascular access device may be implanted in a manner which reduces scarring, and reduces the risk of infection and can be more comfortable for patients.

The tunneler may be used to create a subcutaneous tunnel from the first access site to the second site or *vice versa.* The tunneler may further be configured to release the vascular access device in a location between the first access site and the second access site as will be explained in more detail with reference to figures 3A and 3B.

The second access site may be in an armpit. With examples of the present disclosure, the second access site may be chosen in different areas than in prior art methods. With less scarring, the second access site may be in area that is more visible and which may be more convenient for the surgeon.

As mentioned before, the tunneler 100 may be configured to be releasably coupled at the second end 20 of the vascular access device 10, whereas the catheter 80 is coupled at the first end 30. The vascular access device 10 has a device side connector to releasably couple to the tunneler. The device side connector may be an eye or eyelet configured to receive a hook 110 of the delivery system. The hook may be opened or closed by a medical professional carrying out the implantation of the device in order to unhook, respectively hook the tunneler 100 to the vascular access device 10.

In the illustrated example, the hook may comprise two parts, which when closed form a closed ring to retain an eyelet of the vascular access device. The two parts are separable to open the ring, such that the eyelet can be removed.

In further examples, the device side connector may be a connector for magnetically coupling to the connector of the delivery system. Either the tunneler or the device side connector may have an electromagnet to actively couple the device to the tunneler, and when the device is in place, uncoupling can occur.

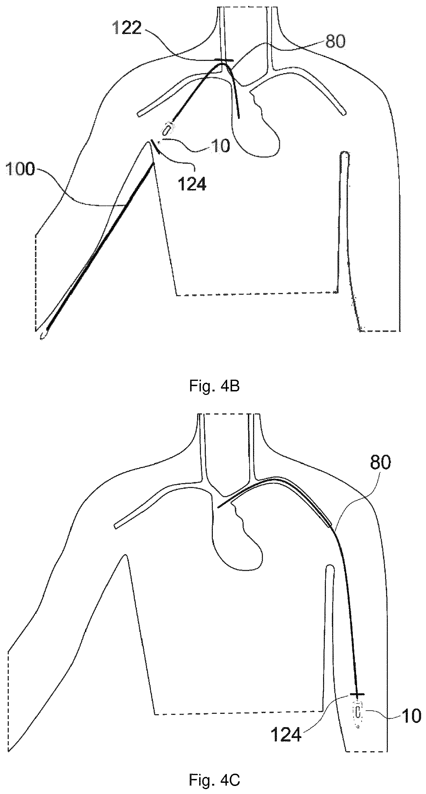

Figures 4A and 4B schematically illustrate an example of a method for delivering a vascular access device 10 to an implantation site. The method comprises providing a first access site 122 through a skin of a patient and introducing a catheter 80 in a vein through the first access site 122.

The vein in which the catheter 80 may be introduced may be any suitable vein, particularly any of External Jugular Vein, Internal Jugular Vein, Cephalic Vein, Subclavian Vein and Femoral Vein.

The method may further comprise providing a second access site 124 through the skin of the patient and providing a tunnel between the first access site and the second access site using a tunneler 100.

As mentioned before, the numbering of the access site as "second" doesn't mean that the second access sire is created after the first. A tunnel may be made by introducing the tunneler 100 from the second access site to the first access site 122.

As mentioned before, variants of the Seldinger technique may be used to position one end of the catheter 80 in the vein. Providing the first access site may comprise a puncture with a hollow needle through the skin and wherein a distal end of the hollow needle reaches the vein, and introduction of a guidewire into the vein through the hollow needle. The catheter may be positioned in the selected vein using further sheaths and/or dilators.

The other end of the catheter may be coupled to the vascular access device, e.g. at connector 35. A suitable length of the catheter 80 may be determined based on the chosen implantation site for the vascular access device 10 and the catheter may be cut to the appropriate length.

The method then comprises releasably coupling the tunneler 100 to the vascular access device 10, and moving the tunneler 100 from the first access site 122 towards the second access site 124 to pull the vascular access device 10 towards the second access site 124. That is, contrary to prior art methods, the vascular access device is introduced into the patient's body at the first access site 122 (the same access site for positioning the catheter in the vein), rather than at the second access site 124.

The incision at the second site may be smaller than in the prior art. The incision at the second site may be smaller than 0,5 cm in length, specifically smaller than 3 mm in length, and more specifically 2 mm or smaller in length.

As the vascular access device 10 is pulled by the tunneler, the method then comprises releasing the vascular access device 10 in the implantation site. The tunneler may then be pulled further to exit through the second access site and the vascular access device is left in place.

In some examples, providing the tunnel between the first access site 122 and the second access site 124 and pulling the vascular access device 10 towards the second access site 124 may be performed simultaneously, i.e. the tunnel is created as the vascular access device 10 is pulled due to its self-dissecting nature.

In other examples, a tunnel is provided from the first access site to the second access site using the tunneler 100, and subsequently the tunneler 100 is releasably coupled to the vascular access device, and the tunneler is moved from the first access site to the second access site.

The method may further comprise visualization of the vascular access device and/or the catheter while the vascular access device is pulled from the first access site to the second access site. Visualization may comprise visualization using X-rays to ensure the catheter is and remains positioned correctly. The catheter may include radiopaque markers in suitable locations. In some examples also vascular access device may include radiopaque markers for visualization using X-rays. As the vascular access device is pulled some traction may exerted on the catheter. X-ray visualization may be used to ensure that the catheter stays positioned in the right location. The ability to correctly position the vascular access device in the correct location is especially important in children, because children's body size varies significantly with age.

The visualization system of the vascular access device to visualize the injection area may also be used during implantation such that it is clear where future puncturing is to take place.

Figure 4C illustrates an alternative example of a method for implanting a vascular access device. As has been explained throughout the present disclosure, the vascular access device may be smaller and thinner than prior art devices. Devices of relatively small dimensions may be implanted in a patient's arm as well. One aspect of implanting the device in the arm of a patient is that such a procedure may be carried out with local anesthesia only and without the need for a fully equipped Operating Room. The Seldinger technique may be used to access the veing and the catheter may be advanced in the deep veins in the middle of the arm.

The catheter may be coupled at an end of the vascular access device as illustrated in previous examples. The introduction of the catheter and the vascular access device may be carried out with a single incision in the arm.

For completeness, a number of aspects of the present disclosure are set out in the following numbered clauses:
Clause 1. An implantable vascular access device comprising:
   a housing;
   a reservoir with an exit lumen;
   a septum for closing the reservoir and defining an injection area;
   a visualization system for visualizing the injection area;
   a controller configured to activate the visualization system, and wherein
   the implantable vascular access device is self-dissecting.
Clause 2. The device according to clause 1, wherein the injection area has a size of 75 mm² or more, specifically 85 mm² or more, more specifically 95 mm² or more.
Clause 3. The device according to clause 1 or 2, wherein a height of the device is less than 10 mm, specifically less than 8 mm, and more specifically 6 mm or less.
Clause 4. The device according to any of clauses 1 - 3, wherein the housing comprises a tapered portion, and optionally wherein the tapered portion has a length of at least 15 mm, specifically more than 30 mm.
Clause 5. The device according to any of clauses 1 - 4, configured not to be palpable through a skin of the patient.
Clause 6. The device according to any of clauses 1 - 5, wherein the injection area is substantially obround.
Clause 7. The device according to any of clauses 1 - 6, wherein the visualization system comprises one or more light emitting devices arranged along a perimeter of the injection area.
Clause 8. The device according to any of clauses 1 - 7, wherein the visualization system comprises an augmented reality device.
Clause 9. The device according to any of clauses 1 - 8, wherein the visualization system is configured to indicate a perimeter of the injection area.
Clause 10. The device according to any of clauses 1 - 9, further comprising an energy source, optionally a battery.
Clause 11. The device according to any of clauses 1 - 10, wherein the controller is configured to activate the visualization system after receiving an instruction from a remote control.
Clause 12. The device according to any of clauses 1 - 11, wherein the visualization system is configured to be active for a predetermined period of time, and optionally wherein the visualization system is configured to indicate a puncture site within the injection area.
Clause 13. The device according to any of clauses 1 - 12, wherein the exit lumen is provided at a first end of the device, and the device further comprising a device side connector for releasably coupling to a connector of a delivery system.
Clause 14. The device according to clause 13, wherein the device side connector is an eye or eyelet configured to receive a hook of the delivery system.
Clause 15. The device according to clause 13, wherein the device side connector is a connector for magnetically coupling to the connector of the delivery system.
Clause 16. The device according to any of clauses 1 - 15, further comprising one or more sensors for measuring patient parameters.
Clause 17. The device according to any of clauses 1 - 16, further comprising an emitter for sending data to a receiver.
Clause 18. A system comprising the device according to clause 16 or 17, further comprising a data processing system comprising the receiver for receiving the data sent by the transmitter of the device and a database for storing the received data.
Clause 19. The system according to clause 18, further comprising a display for visualizing the received data.
Clause 20. The system of clause 18 or 19, wherein the data processing system is a personal computer, a tablet, a mobile phone, a Smartphone, or a personal digital assistant.
Clause 21. The system of any of clauses 18 - 20, wherein the data processing system is further configured to send one or more instructions to the controller of the implantable vascular access device.
Clause 22. A kit comprising the implantable vascular access device according to any of clauses 1 - 17, and a remote control for sending one or more instructions to the controller of the implantable vascular access device.
Clause 23. The kit according to clause 22, wherein the remote control is a personal computer, a tablet, a mobile phone, a Smartphone, or a personal digital assistant.
Clause 24. The kit according to clause 22, wherein the remote control is integrated in a needle.
Clause 25. An implantable vascular access device comprising:
   a housing;
   a reservoir with an exit lumen;
   a septum for closing the reservoir and defining an injection area;
   one or more sensors arranged to measure one or more parameters of blood in the reservoir; and
   a transmitter for sending the measured parameters.
Clause 26. The device according to clause 25, wherein the sensors are configured to measure one or more of: a temperature, cardiac rhythm, oxygen saturation, glycaemia.
Clause 27. The device according to clause 25 or 26, further comprising a visualization system for visualizing the injection area and a controller configured to activate the visualization system.
Clause 28. The device according to clause 27, wherein the visualization system comprises one or more light sources configured to indicate a perimeter of the injection area.
Clause 29. The device according to any of clauses 25 - 28, wherein the housing comprises a tapered portion comprising the transmitter, and an electronics support, and optionally wherein the tapered portion has a length of 15 mm or more, and optionally wherein the tapered portion has one or more taper angles is 35º or less.
Clause 30. The device according to any of clauses 25 - 29, configured not to be palpable through a skin of the patient.
Clause 31. The device according to any of clauses 25 - 30, wherein the injection area is substantially obround.
Clause 32. The device according to any of clauses 25 - 31, wherein the outer contour of the device is substantially smooth.
Clause 33. A system comprising the device according to any of clauses 25 - 32, further comprising a data processing system including a receiver for receiving the parameters sent by the transmitter.
Clause 34. The system according to clause 33, wherein the data processing system is a personal computer, a tablet, a mobile phone, a Smartphone, or a personal digital assistant.
Clause 35. The system of clause 33 of 34, wherein the data processing system is further configured to send one or more instructions to the controller of the implantable vascular access device.
Clause 36. A method for locating a vascular access device implanted in a patient, comprising:
   remotely activating a visualization system of the vascular access device to visualize a perimeter of an injection area of the vascular access device, and
   locating the vascular access device by detecting the perimeter of the injection area while refraining from palpating the patient.
Clause 37. The method according to clause 36, wherein remotely activating the visualization system comprises sending an activation signal from one or more of a personal computer, a tablet, a mobile phone, a Smartphone, or a personal digital assistant.
Clause 38. The method according to clause 36 or 37, wherein the visualization system is configured to visualize the perimeter of the injection area during a predetermined period of time.
Clause 39. The method according to any of clauses 36 - 38, wherein a height of the vascular access device is less than 10 mm, specifically less than 8 mm, and more specifically 6 mm or less.
Clause 40. The method of any of clauses 36 - 39, wherein the vascular access device is not visible from an outside of the patient when the visualization system is not activated.
Clause 41. A delivery system for delivering a vascular access device, and particularly a self-dissecting vascular access device,
   the vascular access device having a first end and a second end, and an exit lumen with a device side connector for coupling to a catheter arranged at the first end, the delivery system comprising:
   a tunneler configured to provide a tunnel between a first access site and a second access site, wherein the tunneler is further configured to be releasably coupled to the vascular access device at the second end of the vascular access device.
Clause 42. The delivery system of clause 41, wherein the tunneler is configured to release the vascular access device in a location between the first access site and the second access site.
Clause 43. The delivery system of clause 41 or 42, wherein the first access site is in or near a neck or infraclavicular region of the patient.
Clause 44. The delivery system of clause 43, wherein the second access site is in an armpit region of the patient.
Clause 45. A method for delivering a vascular access device, particularly a self-dissecting vascular access device, to an implantation site:
   providing a first access site through a skin of a patient;
   introducing a catheter in a vein through the first access site;
   providing a second access site through the skin of the patient;
   providing a tunnel between the first access site and the second access site using a tunneler;
   releasably coupling the tunneler to the vascular access device, and moving the tunneler from the first access site towards the second access site to pull the vascular access device towards the second access site;
   releasing the vascular access device in the implantation site.
Clause 46. The method of clause 45, wherein providing the tunnel between the first access site and the second access site and pulling the vascular access device towards the second access site is performed simultaneously.
Clause 47. The method of clause 45, wherein a tunnel is provided from the first access site to the second access site using the tunneler, and wherein subsequently the tunneler is releasably coupled to the vascular access device, and the tunneler is moved from the first access site to the second access site.
Clause 48. The method of any of clauses 45 - 47, wherein providing the first access site comprises a puncture with a hollow needle through the skin and wherein a distal end of the hollow needle reaches the vein, and introduction of a guidewire into the vein through the hollow needle.
Clause 49. The method of any of clauses 45 - 48, wherein providing the second access site comprises making an incision in the skin, wherein the incision is smaller than 0,5 cm in length, specifically smaller than 3 mm in length, and more specifically 2 mm or smaller in length.
Clause 50. The method of any of clauses 45 - 49, wherein the tunneler is releasably mechanically coupled to the vascular access device.
Clause 51. The method of clause 50, wherein the tunneler comprises a hook for coupling with an eye or eyelet of the vascular access device.
Clause 52. The method of any of clauses 45 - 51, further comprising visualization of the vascular access device and/or the catheter while the vascular access device is pulled from the first access site to the second access site.
Clause 53. The method of clause 52, wherein visualization comprises visualization using X-rays.
Clause 54. The method of any of clauses 45 - 53, wherein a height of the vascular access device is less than 10 mm, specifically less than 8 mm, and more specifically 6 mm or less.
Clause 55. The method of any of clauses 45 - 54, wherein the vascular access device comprises one or more sensors arranged to measure one or more parameters of blood in the reservoir and a transmitter for sending the measured parameters.
Clause 56. The method of any of clauses 45 - 55, wherein the vascular access device comprises a tapered portion.
Clause 57. The method of clause 56, wherein the tapered portion has a length of 15 mm or more, specifically 30 mm or more.
Clause 58. The method of clause 56 or 57, wherein the tapered portion has one or more taper angles of 35º or less, specifically 20º or less.

Although only a number of examples have been disclosed herein, other alternatives, modifications, uses and/or equivalents thereof are possible. Furthermore, all possible combinations of the described examples are also covered. Thus, the scope of the present disclosure should not be limited by particular examples, but should be determined only by a fair reading of the claims that follow.

## Claims

1. An implantable vascular access device comprising:
a housing;
a reservoir with an exit lumen;
a septum for closing the reservoir and defining an injection area;
a visualization system for visualizing the injection area;
a controller configured to activate the visualization system, and wherein
the implantable vascular access device is self-dissecting.

2. The device according to claim 1, wherein a height of the device is less than 10 mm, specifically less than 8 mm, and more specifically 6 mm or less.

3. The device according to claim 1 or 2, configured not to be palpable through a skin of the patient.

4. The device according to any of claims 1 - 3, further comprising a tapered portion, and optionally wherein the taper angle is smaller than 35°.

5. The device of claim 4, wherein the tapered portion has a length of at least 15 mm, specifically more than 30 mm.

6. The device according to any of claims 1 - 5, wherein the injection area has a size of 75 mm² or more, specifically 85 mm² or more, more specifically 95 mm² or more.

7. The device according to any of claims 1 - 6, wherein the visualization system comprises one or more light emitting devices arranged along a perimeter of the injection area.

8. The device according to any of claims 1 - 7, wherein the controller is configured to activate the visualization system after receiving an instruction from a remote control.

9. The device according to any of claims 1 - 8, further comprising one or more sensors for measuring patient parameters.

10. The device according to claim 9, wherein the sensors are configured to measure one or more of: a temperature, cardiac rhythm, oxygen saturation, glycaemia.

11. The device according to any of claims 1 - 10, further comprising an emitter for sending data to a receiver.

12. The device according to any of claims 1 - 11, further wherein the exit lumen is provided at a first end of the device, and the device further comprising a device side connector for releasably coupling to a connector of a delivery system.

13. A delivery system for delivering a vascular access device according to claim 12, the vascular access device having a first end and a second end, and an exit lumen with a device side connector for coupling to a catheter arranged at the first end, the delivery system comprising:
a tunneler configured to provide a tunnel between a first access site and a second access site, wherein
the tunneler is further configured to be releasably coupled to the vascular access device at the second end of the vascular access device.

14. The delivery system of claim 13, wherein the tunneler is configured to release the vascular access device in a location between the first access site and the second access site.

15. A system comprising the device according to any of claims 1 - 12, further comprising a data processing system comprising the receiver for receiving the data sent by the transmitter of the device and a database for storing the received data, wherein the data processing system optionally is a personal computer, a tablet, a mobile phone, a Smartphone, or a personal digital assistant.
